# FASCICULE DE BREVET EUROPEEN

(11) **EP 2 335 637 B1**
(45) Date de publication et mention de la délivrance du brevet: **17.08.2016**
(21) Numéro de dépôt: 10192933.9
(22) Date de dépôt: 29.11.2010
(51) Int. Cl.: A61C 1/00, A61C 19/08, A61J 1/16, A61M 5/24, B65D 23/08, B65D 77/04

(54) **Dispositif d'injection avec support cylindrique pour un contenant de produit pharmaceutique destiné à être mis en rotation**
Injektionsvorrichtung mit zylindrischer Halterung für rotierbare Medikamentenampulle
Injection device with cylindrical support for rotatable drug reservoir

(30) Priorité: 17.12.2009 FR 0959141
(43) Date de publication de la demande: 22.06.2011
(73) Titulaire: Dentalhitec, 49280 Mazieres en Mauges (FR)
(72) Inventeur: Villette, Olivier, 49600, ANDREZE (FR); Ripaud, Denis, 49230, TILLIERES (FR)
(74) Mandataire: Thinat, Michel

(56) Documents cités:
- EP-A1- 2 022 522
- WO-A2-2006/007590
- WO-A2-2007/018809
- FR-A- 822 764
- FR-A- 1 238 213
- FR-A1- 2 138 157
- FR-A1- 2 540 385
- GB-A- 724 367
- US-A- 1 718 591
- US-A- 5 590 782
- US-A1- 2009 107 947
- US-B1- 6 245 043

## Description

La présente invention concerne un instrument de chirurgie, pour la perforation d'un corps humain ou animal et l'injection d'un produit pharmaceutique dans ce corps, qui comprend un support cylindrique pour un contenant de produit pharmaceutique, destiné à être mis en rotation, selon le préambule de la revendication 1.

Certaines applications médicales, par exemple dans le domaine de l'anesthésie dentaire, nécessitent de traverser des tissus denses, par exemple la corticale osseuse d'une mâchoire, pour pouvoir injecter un produit pharmaceutique au bon endroit. Pour y arriver, on réalise d'abord une perforation avec un foret puis on fait l'injection avec une aiguille. Selon une méthode alternative, on réalise la perforation avec une aiguille perforante servant aussi à l'injection.

Dans le cas d'une utilisation de l'aiguille pour la perforation, le moyen le plus simple et qui nécessite le moins de place est d'utiliser le contenant de produit anesthésique comme arbre de transmission entre des moyens d'entraînement et l'aiguille. Il faut donc concevoir une pièce d'adaptation capable de transmettre le couple du contenant de produit pharmaceutique à l'aiguille perforante. La pièce d'adaptation doit être parfaitement coaxiale avec le contenant pour supporter les vitesses de rotation élevées sans engendrer de vibrations ni de bruit important. De plus, la pièce d'adaptation doit être capable d'être stérilisée à des températures élevées (entre 130 et 140°C), car l'aiguille perforante et souvent aussi l'extrémité de la pièce d'adaptation recevant l'aiguille, sont en contact avec le patient, notamment dans la bouche de celui-ci.

Les pièces d'adaptation utilisées avant l'invention sont réalisées dans des matériaux plastiques qui utilisent la déformation du matériau pour enserrer le contenant et pour transmettre ainsi le couple à l'aiguille perforante. Toutefois, les matériaux utilisés se sont avérés désavantageux dans la mesure où ils font preuve d'un vieillissement prématuré dû à des contraintes internes fortes pour s'adapter aux différents diamètres des contenants. Ces contraintes internes fortes ne permettent pas d'emmanchement sur des longueurs importantes, ce qui ne favorise pas la coaxialité de la rotation du contenant par rapport à l'aiguille.

D'autres supports en plastique ont une longueur proche de celle des contenants et comportent, pour s'adapter aux différents diamètres des contenants, des bagues en caoutchouc, donc des joints internes annulaires logés dans des rainures annulaires prévues à cet effet dans les supports.

Cependant, ces supports se sont avérés désavantageux par la mauvaise résistance dans le temps des produits caoutchoucs confrontés lors de la stérilisation à des températures entre 130°C et 140°C et aux produits de nettoyage. De plus, les joints en caoutchouc provoquent une augmentation du diamètre extérieur du support. Cette augmentation entraîne une augmentation du diamètre de la pièce à main, ce qui présente un problème important dans la mesure où l'on cherche toujours la maniabilité maximale, la vision optimale et le moindre encombrement.

En ce qui concerne les contenants en verre, ils présentent, par leur mode de fabrication, des variations importantes sur leur diamètre et leur longueur. Certains modèles reçoivent en plus un film plastique anti-fracture ce qui augmente encore les variations de diamètre et modifie la surface de contact qui glisse mal sur les joints en caoutchouc.

Le but de l'invention est donc de proposer les moyens qui permettent de remédier aux désavantages énoncés ci-avant.

Des supports cylindriques adaptés pour y emmancher un contenant cylindrique sont connus dans l'état de la technique. Ainsi, le document US-A-2009/0107947 décrit un manchon en élastomère conçu pour servir comme protection d'un contenant en verre, par exemple une bouteille. Ce manchon en élastomère assure un contact permanent avec le contenant en le serrant grâce à sa capacité élastique. Cependant, la liaison entre le manchon en élastomère et le contenant n'a pas à être suffisamment forte pour assurer une immobilité en rotation entre le contenant et le manchon.

Le document EP-A-2 022 522 décrit un instrument de chirurgie contenant un support cylindrique avec une aiguille d'injection perforante qui est connue pour recevoir un contenant de produit pharmaceutique et qui est entraîné en rotation par des moyens agissant sur sa périphérie. Dans cette configuration, le contenant de produit pharmaceutique n'est pas utilisé comme arbre de transmission de la force d'entraînement en rotation.

Le document WO-A-2006/007590 décrit un instrument de chirurgie avec l'aiguille perforante où l'aiguille est entraînée directement et c'est elle qui entraîne le contenant des produits pharmaceutiques.

Les documents US 6 245043, FR 2 540 385 et WO 2007 018809 décrivent d'autres dispositifs d'injection avec des contenants et aiguilles pouvant être mis en rotation.

Le but de l'invention est atteint avec un instrument de chirurgie comportant les caractéristiques énoncées dans la partie caractérisante de la revendication 1.

Des variantes avantageuses sont décrites dans les revendications dépendantes.

Le support cylindrique selon l'invention peut être réalisé avec des matériaux plastiques qui supportent très bien la stérilisation et les produits de nettoyage. Le fait que ces matériaux sont souvent très rigides est compensé par le fait que le support cylindrique a une forme intérieure spécifique qui crée une zone de contact avec le contenant aux extrémités, ou à proximité de chacune des extrémités, du support cylindrique pour permettre une coaxialité optimale. Les zones de contact sont formées par des ponts de matière subsistants entre des évidements pratiqués le long de lignes de rangées circonférentielles, les ponts de matière présentant des fléchissements vers l'intérieur du support cylindrique.

Grâce à cette disposition de l'invention, on donne au corps tubulaire du support cylindrique une certaine élasticité qui lui permet de s'adapter, en permanence, aux variations de diamètre des contenants anesthésiques utilisés et de répartir la déformation de manière équivalente sur les ponts de matière. En même temps, le support cylindrique peut transmettre un couple suffisant, dans un volume minimal et supporte bien les contraintes de nettoyage et de stérilisation. Lorsque le contenant est introduit dans le support cylindrique, les fléchissements des ponts de matière se réduisent de manière élastique et lorsque le contenant est retiré, les fléchissements reprennent leur forme initiale.

La description est faite en référence aux dessins dans lesquels :
- la figure 1 représente un instrument de chirurgie selon l'invention,
- la figure 2 représente un support cylindrique avec une cartouche en tant que contenant d'un produit pharmaceutique en état d'assemblage,
- la figure 3 représente le support cylindrique et la cartouche séparée,
- la figure 4 représente un support cylindrique en vue latérale,
- la figure 5 représente un support cylindrique en une vue en perspective du côté de l'ouverture axiale et
- la figure 6 représente le support des figures 4 et 5 en une vue en coupe axiale avec un contenant engagé dans le support cylindrique.

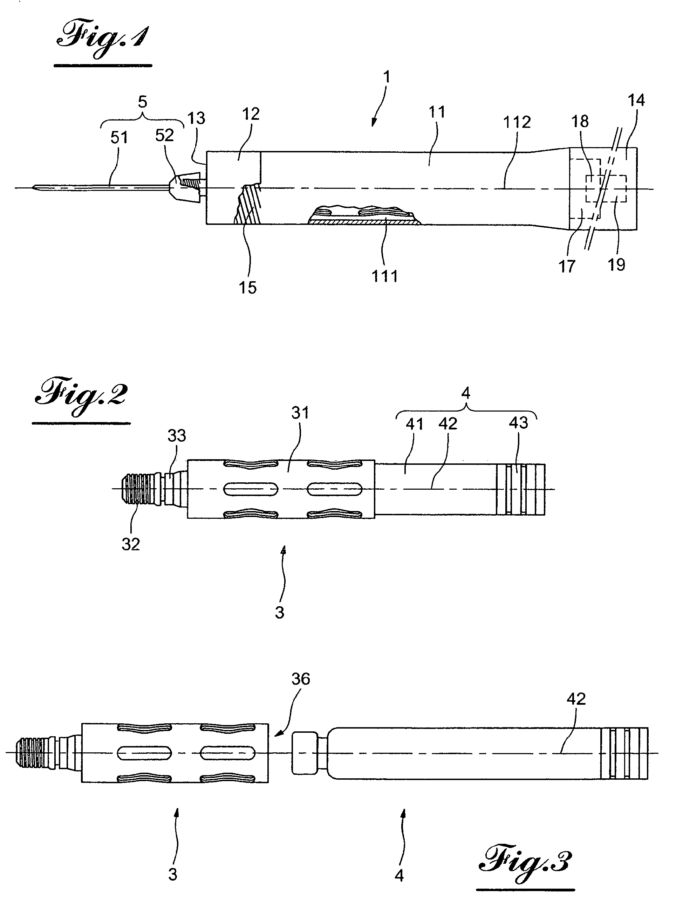

La figure 1 représente, en une vue schématique latérale avec un arrachement, un instrument de chirurgie selon l'invention.

L'instrument comprend une pièce à main 1 ayant un corps allongé 11 avec un logement 111 et deux extrémités opposées 12, 14. L'extrémité 12 est formée par une tête amovible pourvue d'un passage axial 13. La tête 12 est avantageusement conformée pour être fixée sur le corps 11 par vissage, le corps 11 étant pourvu à cet effet d'un filetage 15 et la tête 12 d'un filetage correspondant. Toutefois, la tête 12 peut tout aussi bien être fixée par clipsage ou encore être intégrée au corps 11 pour ne faire qu'une seule pièce. L'extrémité 14 de la pièce à main 1 est formée par une pièce de fond montée par clipsage ou, selon une variante de réalisation non représentée, par vissage sur un raccord 17 du corps 11.

Le corps 11 de la pièce à main 1 est conformé pour recevoir, dans le logement 111 qui est symétrique en rotation autour d'un axe 112, un support cylindrique 3 et un contenant 4 de produit pharmaceutique, le produit pharmaceutique étant une solution injectable. Le contenant 4, également appelé une cartouche, comprend un corps cylindrique de rotation 41 avec un axe 42 et un piston 43 pour pouvoir agir sur le contenu. En état d'utilisation, le contenant 4 est emmanché dans le support cylindrique 3, comme représenté sur la figure 2.

Le support cylindrique 3 est un élément allongé creux avec un goulot pourvu d'une partie filetée 32 à l'une de ses deux extrémités opposées et avec une ouverture axiale 36 pour l'emmanchement du contenant 4 à l'autre extrémité. Le support 3 est formé de façon que le goulot soit logé dans le passage axial 13 de la tête 12 de la pièce à main 1 et dépasse de celle-ci vers l'extérieur par la partie filetée 32 du goulot afin de pouvoir recevoir une aiguille perforante 5 par vissage. L'aiguille 5 comporte à cet effet, outre une canule 51, un embout de fixation fileté 52.

La pièce de fond 14 retient le support cylindrique 3 à l'intérieur du corps allongé 11 et le maintient en appui sur une partie intérieure de la tête 12 conformée à cet effet. En outre, la pièce de fond 14 comprend un organe 18 conformé pour être en appui sur un piston 43 du contenant 4 afin de pouvoir exercer une pression sur le produit pharmaceutique contenu dans le contenant 4. L'organe 18 est configuré pour pouvoir non seulement injecter une dose prévue du produit pharmaceutique dans le corps humain ou animal mais aussi pour introduire et maintenir sous pression, en une ou plusieurs fois, le produit pharmaceutique dans l'aiguille 5 selon des critères de fonctionnement décrits plus loin dans la présente description. Dans la pièce de fond 14 sont également logés des moyens d'entraînement 19 permettant de mettre l'ensemble support/contenant en rotation autour de l'axe longitudinal 42 du corps 41 du contenant 4.

Les figures 2 et 3 représentent respectivement en état assemblé et en état séparé le support cylindrique 3 et le contenant de produit pharmaceutique 4. Le support cylindrique 3 comporte un corps allongé creux ou tubulaire 31 ayant un axe longitudinal 30. Le corps 31 est réalisé en un matériau plastique rigide et est fermé à une de ses deux extrémités opposées par le goulot mentionné déjà plus haut. A l'autre extrémité, le corps 31 est pourvu d'une ouverture axiale 36 pour l'emmanchement d'un contenant 4. Le goulot comprend une partie filetée 32 pour porter l'aiguille perforante 5 et est muni d'une lumière par laquelle l'aiguille 5 pénètre d'abord à l'intérieur du corps 31 et ensuite traverse la membrane élastique du contenant 4 pour aboutir à l'intérieur de ce dernier. Ainsi, en appuyant sur le piston 43, le produit pharmaceutique sort du contenant 4 par l'aiguille 5 et est injecté dans un corps humain ou animal. Le goulot est également pourvu d'une piste de roulement circonférentielle 33 destinée à, et conformée pour, coopérer avec un roulement mécanique (non représenté) disposé dans la tête 12 de la pièce à main 1 et destiné à réduire la résistance à la rotation lorsque l'instrument de chirurgie est utilisé en mode de perforation.

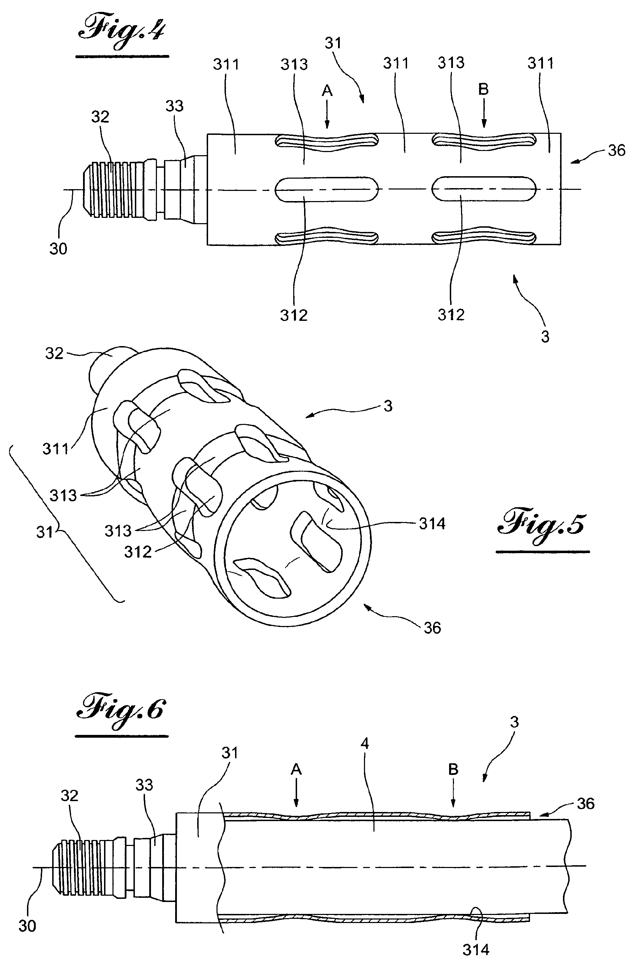

Les figures 4 et 5 représentent le support cylindrique 3 seul respectivement en une vue latérale et en une vue en perspective. Le corps 31 du support cylindrique 3 est essentiellement un élément tubulaire à diamètre extérieur constant sur la longueur axiale entière du corps 31, comme en témoignent des parties 311 à paroi pleine. L'exception à la caractéristique de diamètres extérieur et intérieur constants est faite dans deux parties du corps 31 où des évidements et des ponts de matière sont disposés en alternance en deux rangées circonférentielles A, B.

En effet, afin de pouvoir donner au corps 31 une certaine élasticité, nécessaire pour pouvoir enserrer un contenant 4, le corps 31 est pourvu d'évidements 312 formés à des distances circonférentielles au moins approximativement égales sur le pourtour entier du corps 31 et en au moins une rangée circonférentielle, de préférence en deux rangées circonférentielles A, B. Les évidements 312 se présentent ici sous forme de trous oblongs s'étendant suivant des axes parallèles à l'axe longitudinal 30 du support cylindrique 3.

Entre les évidements 312 d'une même rangée subsistent des ponts de matière 313 fléchis vers l'intérieur du corps tubulaire 31 afin de pouvoir coopérer élastiquement avec un contenant 4 ainsi enserré dans le corps 31. Les ponts de matière 313 sont disposés de façon à saisir le contenant 4, lorsque celui-ci est introduit dans le support 3, proche des extrémités du support 3 pour obtenir la plus grande stabilité axiale du contenant 4 par rapport à l'aiguille de perforation 5.

Comme la figure 6 le montre dans une représentation en perspective avec vue sur l'ouverture 36 du support cylindrique 3, le fléchissement de l'intérieur des ponts de matière 313 crée des parties en pente 314 intérieures. Lorsqu'un contenant 4 est introduit dans un support cylindrique 3, le contenant 4 prend appui sur les pentes 314 des ponts de matière 313 et les écarte radialement les uns des autres à l'encontre de la force exercée par les ponts de matière 313. Cette disposition assure une assise permanente et coaxiale du contenant 4 par rapport au support cylindrique 3. En même temps, lorsque le contenant 4 est retiré du support cylindrique 3, les ponts de matière 313 fléchissent vers l'intérieur, c'est-à-dire ils reprennent leur forme initiale de fléchissement.

Cette disposition de l'invention rend superflu l'utilisation de joints en caoutchouc ou d'autres moyens élastiques supplémentaires et assure avec la seule élasticité des ponts de matière 313 une adaptation du support 3 aux différents diamètres extérieurs des contenants 4.

## Revendications

1. Instrument de chirurgie pour la perforation d'un corps humain ou animal et l'injection d'un produit pharmaceutique dans ce même corps, comprenant une pièce à main (1) allongée creuse adaptée pour recevoir un contenant (4) de produit pharmaceutique et un support cylindrique (3) permettant de loger le contenant (4) dans la pièce à main (1), des moyens d'entraînement (19) permettant de mettre l'ensemble support / contenant en rotation autour d'un axe longitudinal (42) du contenant (4), le support cylindrique (3) permettant de relier une aiguille perforante (5) au contenant (4), la pièce à main (1) étant pourvue d'un passage axial (13) pour l'aiguille (5),
**caractérisé en ce que** le support cylindrique (3) comprend une paroi tubulaire (311) adaptée pour recevoir le contenant (4) et comportant des zones de contact pour une liaison solidaire en rotation entre le contenant (4) et le support cylindrique (3) formées par des ponts de matière (313) subsistants entre des évidements (312) pratiqués le long d'au moins une ligne de rangée circonférentielle, les ponts de matière (313) présentant des fléchissements vers l'intérieur du support cylindrique (3) pour enserrer le contenant.

2. Instrument de chirurgie selon la revendication 1, **caractérisé en ce que** le support cylindrique (3) comprend un goulot fileté (32) dans lequel l'aiguille (5) est engagée par vissage pour relier l'aiguille (5) au contenant (4), l'aiguille (5) s'étendant jusqu'à l'intérieur du contenant lorsque celui-ci est placée dans le support cylindrique (3), le support (3) reliant ainsi l'aiguille d'injection (5) au contenant (4).

3. Instrument de chirurgie selon la revendication 1 ou 2, **caractérisé en ce que** le support cylindrique (3) comprend deux rangées circonférentielles (A, B) d'évidements (312) espacées suivant l'axe longitudinal (30) du support cylindrique (3).

4. Instrument de chirurgie selon l'une quelconque des revendications 1 à 3, **caractérisé en ce que** les évidements (312) du support cylindrique (3) sont des trous oblongs orientés parallèlement à l'axe longitudinal (30) du support cylindrique (3).

5. Instrument de chirurgie selon l'une quelconque des revendications 1 à 4, **caractérisé en ce que** le support cylindrique (3) est réalisé en un matériau plastique rigide.

## Patentansprüche

1. Chirurgisches Instrument für das Einstechen eines menschlichen oder tierischen Körpers und die Injektion eines pharmazeutischen Produkts in eben diesen Körper, umfassend ein hohles längliches Handteil (1), das ausgebildet ist, um einen Behälter (4) eines pharmazeutischen Produkts zu empfangen, und einen zylindrischen Halter (3), der erlaubt, den Behälter (4) in dem Handteil (1) unterzubringen, wobei Antriebsmittel (19) erlauben, die Einheit Halter/Behälter um eine Längsachse (42) des Behälters (4) in Rotation zu versetzen, wobei der zylindrische Halter (3) erlaubt, eine Einstechende Nadel (5) mit dem Behälter zu verbinden, wobei das Handteil (1) mit einem axialen Durchgang (13) für die Nadel (5) ausgestattet ist,
**dadurch gekennzeichnet, dass** der zylindrische Halter (3) eine rohrförmige Wand (311) umfasst, die ausgebildet ist, um den Behälter (4) aufzunehmen und Kontaktzonen für eine feste Rotationsverbindung zwischen dem Behälter (4) und dem zylindrischen Halter (3) aufweist, die von verbliebenen Materialbrücken (313) zwischen Aussparungen (312) entlang mindestens einer Umfangsreihenlinie gebildet sind, wobei die Materialbrücken (313) Biegungen in das Innere des zylindrischen Halters (3) aufweisen, um den Behälter einzuspannen.

2. Chirurgisches Instrument nach Anspruch 1, **dadurch gekennzeichnet, dass** der zylindrische Halter (3) einen gewindeten Hals (32) umfasst, in welchen die Nadel (5) durch Schrauben eingeführt ist, um die Nadel (5) mit dem Behälter (6) zu verbinden, wobei sich die Nadel (5) bis in das Innere des Behälters erstreckt, wenn dieser in dem zylindrischen Halter (3) plaziert ist, wobei der Halter (3) dadurch die Injektionsnadel (5) mit dem Behälter (4) verbindet.

3. Chirurgisches Instrument nach Anspruch 1 oder 2,
**dadurch gekennzeichnet, dass** der zylindrische Halter (3) zwei Umfangsreihen (A, B) von Aussparungen (312) umfasst, die gemäß einer Längsachse (30) vom zylindrischen Halter (3) beabstandet sind.

4. Chirurgisches Instrument nach einem der Ansprüche 1 bis 3, **dadurch gekennzeichnet, dass** die Aussparungen (312) des zylindrischen Halters (3) Langlöcher sind, die parallel zur Längsachse (30) des zylindrischen Halters (3) ausgerichtet sind.

5. Chirurgisches Instrument nach einem der Ansprüche 1 bis, **dadurch gekennzeichnet, dass** der zylindrische Halter (3) aus einem starren Kunststoffmaterial hergestellt ist.

## Claims

1. A surgical instrument for perforating a human or animal body and injecting a pharmaceutical product into that same body, comprising a hollow elongated hand piece (1) suitable for receiving a pharmaceutical product container (4) and a cylindrical support (3) making it possible to house the container (4) in the hand piece (1), driving means (19) making it possible to set the support/container assembly in rotation around a longitudinal axis (42) of the container (4), the cylindrical support (3) making it possible to connect a perforating needle (5) to the container (4), the hand piece (1) being provided with an axial passage (13) for the needle (5),
**characterized in that** the cylindrical support (3) comprises a tubular wall (311) suitable for receiving the container (4) and including contact zones for a connection secured in rotation between the container (4) and the cylindrical support (3) formed by material bridges (313) remaining between recesses (312) formed along at least one circumferential row line, the material bridges (313) having bends toward the inside of the cylindrical support (3) to grip the container.

2. The surgical instrument according to claim 1,
**characterized in that** the cylindrical support (3) comprises a threaded neck (32) in which the needle (5) is engaged by screwing to connect the needle (5) to the container (4), the needle (5) extending to the inside of the container when the latter is placed in the cylindrical support (3), the support (3) thus connecting the injection needle (5) to the container (4).

3. The surgical instrument according to claim 1 or 2,
**characterized in that** the cylindrical support (3) comprises two circumferential rows (A, B) of recesses (312) spaced apart along the longitudinal axis (30) of the cylindrical support (3).

4. The surgical instrument according to any one of claims 1 to 3, **characterized in that** the recesses (312) of the cylindrical support (3) are oblong holes oriented parallel to the longitudinal axis (30) of the cylindrical support (3).

5. The surgical instrument according to any one of claims 1 to 4, **characterized in that** the cylindrical support (3) is made from a rigid plastic material.
